# DEMANDE DE BREVET EUROPEEN

(11) **EP 1 964 585 A2**
(43) Date de publication de la demande: **03.09.2008**
(21) Numéro de dépôt: 08447015.2
(22) Date de dépôt: 29.02.2008
(51) Int. Cl.: A61M 5/148

(54) **Appareil autonome et perfusion**

(30) Priorité: 02.03.2007 BE 200700091
(71) Demandeur: CEWAC asbl - Center d'Etudes Wallon De l'Assemblage et du Contrôle des Matériaux, 4102 Ougree-Seraing (BE); Promovet Sarl, 08600 Givet (FR)
(72) Inventeur: Mawet, Pascal, 4910 Polleur (BE); Peeters, Thierry, 4877 Olne (BE); Renard, Philippe, 4190 Xhoris (BE)
(74) Mandataire: Lerho, Marc J. A.

(57) **Abrégé**

La présente invention se rapporte à un appareil autonome de perfusion muni d'un régulateur (10) de la pression exercée sur la poche (5) par la plaque mobile (1), comprenant un ressort (15) ayant un axe perpendiculaire à la plaque mobile (1) et dont une extrémité est couplée à la plaque mobile (1) et l'autre extrémité est reliée indirectement à un appui fixe (14) via une douille contenant un piston (16), attaché d'une part à l'extrémité inférieure du ressort (15) et délimitant d'autre part une chambre de pression (17) formée dans la douille, ladite chambre de pression (17) étant reliée en circuit fermé, par une conduite (21) aboutissant à un orifice (19), à une source de pression constante P_{D} (20), qui exerce sur le piston (16) une force de pression qui compense la diminution de l'effort du ressort (15) sur la plaque mobile (1) au cours de la perfusion, ce qui par conséquent compense la diminution de la force appliquée à la plaque mobile (1) par le mécanisme élastique (2, 3, 6).

## Description

### Objet de l'invention

La présente invention concerne un appareil autonome de perfusion selon le préambule de la revendication 1.

### Etat de la technique

La perfusion est une technique d'intervention, bien connue dans le domaine médical, pour l'injection lente et continue d'un liquide, tel qu'un médicament ou un liquide biologique, dans un organisme. Une telle intervention se pratique couramment, notamment en médecine humaine. Elle est en fait destinée à compenser des pertes de liquide(s) biologique(s), par exemple de sang, subies par un organisme à la suite, entre autres, d'une intervention chirurgicale, d'un accident de la route ou de toute circonstance ayant entraîné de telles pertes. C'est notamment aussi le cas dans des situations de conflit armé. Il est par ailleurs fréquent que l'organisme qui reçoit la perfusion se trouve en état de choc.

Par principe, la perfusion consiste en un écoulement d'un liquide approprié, contenu dans une poche souple, depuis cette poche jusqu'à l'organisme récepteur, via un circuit défini appelé aussi ligne de perfusion. Cet écoulement doit se faire avec un débit aussi constant que possible, adapté aux besoins de l'organisme qui le reçoit.

Selon la pratique habituelle, la poche de liquide est maintenue à une certaine hauteur au-dessus,de l'organisme à perfuser, de façon à assurer un écoulement du liquide par gravité. Cette méthode s'applique assez facilement en milieu hospitalier ou analogue, où la poche peut être suspendue à une hauteur constante grâce à une potence et accompagner le patient dans ses déplacements éventuels. A l'extérieur, par exemple sur les lieux d'un accident de la route ou d'une catastrophe naturelle, le maintien de la poche à la hauteur désirée mobilise un membre du personnel d'intervention, qui n'est dès lors plus disponible pour des missions de soins proprement dites. De plus, il n'est guère possible d'assurer un débit constant de liquide dans ces conditions.

Par ailleurs, particulièrement avec des poches de grandes dimensions, cette méthode ne permet pas toujours de garantir un débit suffisamment constant, même si la poche est maintenue à une hauteur constante, puisque la pression du liquide - et dès lors son débit - diminue au fur et à mesure que la poche se vide. Enfin, il existe un risque non négligeable que le patient, par un geste incontrôlé, arrache la ligne de perfusion qui le relie à la poche suspendue.

Pour tenter de remédier à ces inconvénients, on a déjà proposé différents appareils de perfusion qui peuvent être attachés directement au corps du patient, par un moyen approprié tel qu'une sangle ou une agrafe de ceinture. De tels appareils sont doublement autonomes, d'une part parce qu'ils ne nécessitent pas de moyens extérieurs de support de la poche de perfusion et d'autre part parce qu'ils n'ont pas besoin d'une source d'énergie extérieure, notamment de la gravité, pour effectuer une perfusion.

On connaît, en particulier selon le document EP-A-0 620 747, un appareil autonome de perfusion qui comporte des moyens pour mettre une poche de perfusion sous pression entre deux plaques de compression parallèles. Lorsque cet appareil se trouve dans sa position normale de repos, par exemple rangé dans une armoire, les plaques de compression sont essentiellement horizontales. Pour faciliter la description et la compréhension, on se référera ici, sauf indication contraire, à un appareil placé dans cette position. Pendant la perfusion, la plaque supérieure reste fixe et la plaque inférieure, portant la poche, peut se déplacer verticalement vers la plaque supérieure sous l'action d'un mécanisme élastique. Pour charger une poche dans l'appareil, il faut soulever la plaque supérieure, qui constitue en fait un couvercle rabattable, afin d'avoir accès à la plaque inférieure pour y déposer la poche ; la fermeture de ce couvercle provoque l'armement du mécanisme élastique.

On connaît également, par le document BE-A-1 013 694, un dispositif autonome de perfusion dans lequel une poche de perfusion est mise sous pression entre une plaque inférieure fixe et une plaque supérieure mobile actionnée par un mécanisme élastique. Dans ce dispositif connu, les fonctions de chargement de la poche et d'armement du dispositif sont indépendantes l'une de l'autre, ce qui permet notamment de disposer une poche au préalable dans le dispositif et de ne la mettre sous pression qu'au moment de son utilisation pour une perfusion.

Un inconvénient important des dispositifs autonomes de perfusion connus est qu'ils ne permettent pas toujours d'assurer un écoulement du liquide de perfusion avec un débit constant pendant toute la perfusion. Dans ces appareils, la pression de perfusion est en effet produite par une plaque se déplaçant en translation parallèle sous l'action d'un mécanisme élastique, aussi appelé pantographe, formé par des leviers croisés et des ressorts.

On a par ailleurs constaté que la pression de perfusion diminuait progressivement au cours de l'opération, particulièrement en fin de perfusion, où cette diminution est très importante. Ceci est dû principalement à la non-linéarité de la tangente de l'angle α (alpha) d'ouverture du pantographe lorsque l'angle α augmente, c'est-à-dire lorsque le pantographe se déploie pour appliquer la pression de perfusion. L'angle α est l'angle formé par l'axe d'un levier et l'axe d'un ressort associé. Cette non-linéarité est particulièrement marquée lorsque l'angle α approche de sa valeur finale. Il en résulte une diminution progressive du débit du liquide et souvent même une grande difficulté de vider complètement la poche de perfusion.

### Buts de l'invention

La présente invention vise à procurer un appareil autonome de perfusion, avec lequel il est possible d'une part d'assurer un débit constant du liquide de perfusion pendant toute la durée d'une perfusion et d'autre part de vider aussi complètement que possible la poche de perfusion.

### Principaux éléments caractéristiques de l'invention

Conformément à l'invention, le problème précité est résolu par un appareil autonome de perfusion présentant les caractéristiques des revendications indépendantes. Des formes de réalisation avantageuses de l'invention sont présentées dans les revendications secondaires.

Selon un premier aspect de l'invention, un appareil autonome de perfusion qui comporte un boîtier et des moyens pour mettre une poche de perfusion sous pression entre une plaque fixe et une plaque mobile essentiellement parallèle à ladite plaque fixe et entraînée perpendiculairement à celle-ci par un mécanisme élastique entre une position d'armement et une position de repos, est caractérisé en ce qu'il est muni d'un régulateur de la pression exercée sur la poche par ladite plaque mobile, comprenant un ressort ayant un axe perpendiculaire à la plaque mobile et dont une extrémité est couplée à la plaque mobile et l'autre extrémité est reliée indirectement à un appui fixe via une douille contenant un piston, attaché d'une part à l'extrémité inférieure du ressort et délimitant d'autre part une chambre de pression formée dans la douille, ladite chambre de pression étant reliée en circuit fermé, par une conduite aboutissant à un orifice, à une source de pression P_{D}, qui exerce sur le piston une force de pression qui compense la diminution de l'effort du ressort sur la plaque mobile au cours de la perfusion, ce qui par conséquent compense la diminution de la pression appliquée à la plaque mobile par ledit mécanisme élastique.

Des modes de réalisation avantageux de l'invention comportent en outre en combinaison une ou plusieurs des caractéristiques suivantes :
- le ressort est un ressort de compression hélicoïdal ;
- la face supérieure de la plaque portant la poche - qui est de préférence la plaque mobile - est inclinée par rapport à un plan horizontal, en direction de la sortie de la poche de perfusion ;
- l'angle d'inclinaison γ (gamma) de ladite face supérieure est avantageusement inférieur à 10°, et est de préférence compris entre 3° et 5°. Du fait de cette inclinaison, la force verticale appliquée à la poche peut être décomposée en une composante tangentielle, parallèle à cette face et orientée dans le sens de la pente de la face, et une composante perpendiculaire à la face. Combinée à l'action de la gravité, cette composante tangentielle, qui agit sur le liquide de perfusion, permet d'améliorer sensiblement la vidange de la poche ;
- des moyens de verrouillage de la plaque mobile dans sa position d'armement, ainsi que des moyens pour déverrouiller ladite plaque mobile en vue de permettre son mouvement vers sa position de repos ;
- des moyens de surveillance du degré de vidange de la poche pendant la perfusion, de préférence comprenant au moins une fenêtre graduée, éventuellement disposée dans un panneau vertical de l'appareil, et s'étendant sur une hauteur au moins égale à la course maximale de la plaque mobile.

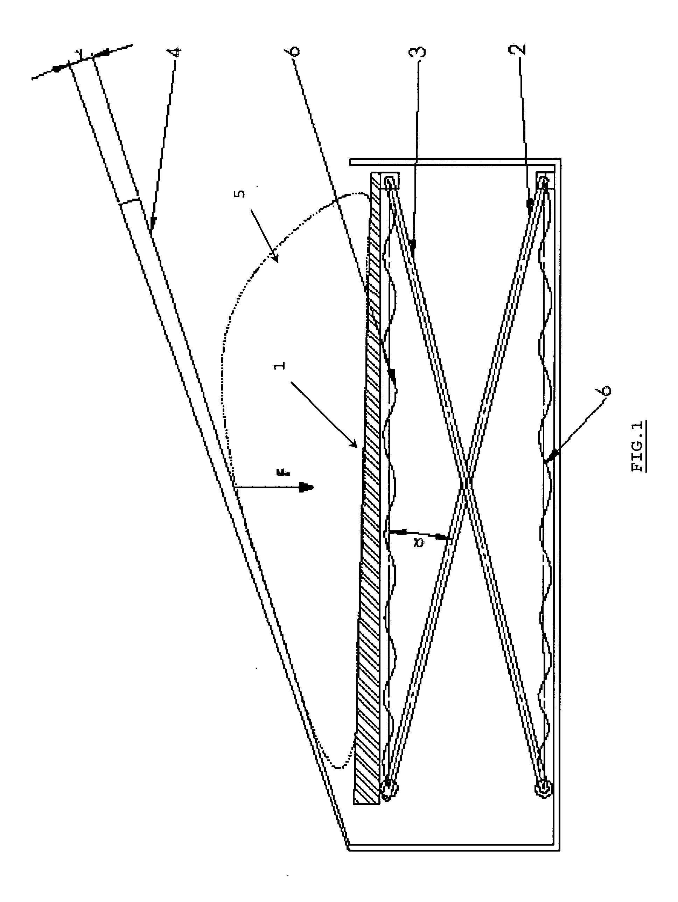

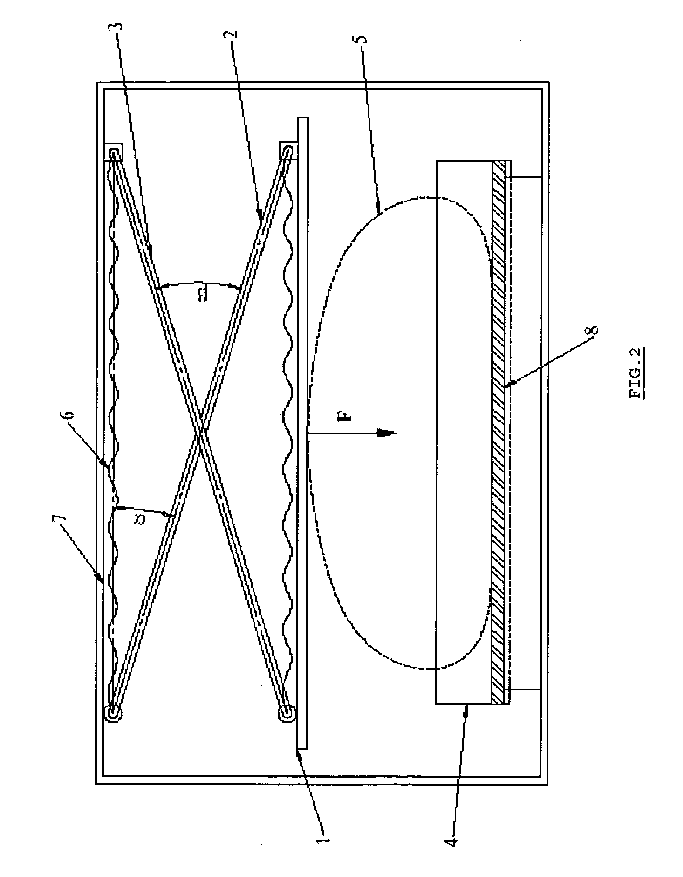

De façon connue en soi, le mécanisme élastique comprend au moins un, de préférence deux ensembles de leviers croisés, parallèles deux à deux, dans lesquels les leviers d'un même ensemble sont réunis par des ressorts et les deux ensembles de leviers sont assemblés par des axes transversaux. Le mécanisme élastique constitue ainsi un pantographe, commandant le déplacement de la plaque mobile entre sa position d'armement et sa position de repos sous l'effet de la traction des ressorts, qui provoque une variation de l'angle d'ouverture α du pantographe. Une description plus détaillée de ce mécanisme élastique et de son mode de fonctionnement sera donnée plus loin en relation avec les figures 1 et 2.

Il convient de préciser qu'au sens de la présente invention, la position d'armement de la plaque mobile est la position dans laquelle elle se trouve lorsque le mécanisme élastique présente son angle d'ouverture minimum et où les ressorts sont tendus, donc avant de commencer la perfusion. La position de repos de la plaque mobile est la position dans laquelle le mécanisme élastique présente son angle d'ouverture maximum et où les ressorts sont détendus, par exemple lorsque la poche est vide.

Comme on l'a indiqué dans l'introduction, l'appareil autonome de perfusion est destiné à être placé notamment sur le corps d'un patient. Il convient dès lors que l'appareil présente des dimensions compatibles avec celles de l'utilisateur.

Il a ainsi été trouvé avantageux que le boîtier de l'appareil soit de forme essentiellement parallélépipédique et présente une longueur de 150 à 300 mm, une largeur de 100 à 200 mm et une hauteur de 40 à 80 mm. Ces dimensions permettent à l'appareil de recevoir les poches de perfusion les plus utilisées actuellement.

Selon un deuxième aspect de l'invention, l'appareil autonome de perfusion comporte un boîtier et des moyens pour mettre une poche de perfusion sous pression entre une plaque fixe et une plaque mobile essentiellement parallèle à ladite plaque fixe et entraînée perpendiculairement à celle-ci par un mécanisme élastique entre une position d'armement et une position de repos, et est caractérisé en ce qu'il est muni d'une source extérieure de pression alimentant, via un régulateur de pression, un vérin souple appuyant sur la plaque mobile, ces éléments étant configurés et agencés pour augmenter au cours de la perfusion la pression exercée par le vérin souple sur la poche via la plaque mobile, cette augmentation de pression s'opposant à la diminution de la pression appliquée à la plaque mobile par ledit mécanisme élastique et par conséquent à la diminution de la pression de sortie de la poche au cours de la perfusion.

Une façon particulièrement avantageuse de mettre en oeuvre cet aspect de l'invention est de prévoir un régulateur de pression qui comprend :
- un cadre rigide essentiellement rectangulaire composé de deux tiges parallèles l'une à l'autre, fixées perpendiculairement à la plaque mobile par une première extrémité et reliées l'une à l'autre, à leur seconde extrémité, par une traverse parallèle à la plaque mobile ;
- un ressort hélicoïdal reliant à son extrémité supérieure la traverse et à son autre extrémité un appui fixe prévu dans le boîtier de l'appareil de perfusion, entre la plaque mobile et la traverse, l'axe longitudinal du ressort étant parallèle aux tiges et donc perpendiculaire à la plaque mobile, la liaison du ressort à l'appui fixe étant réalisé indirectement via une douille contenant un piston, attaché d'une part à l'extrémité inférieure du ressort et délimitant d'autre part une chambre de pression formée dans la douille ;
- une première conduite reliant la chambre de pression à la sortie de la source de pression P_{D} et une seconde conduite reliant la chambre de pression au vérin souple, qui comprend un ballonnet gonflable, appuyant sur la plaque mobile ;
- des orifices calibrés dans le régulateur, par lesquels, lors du déplacement du piston vers le haut sous l'effet de ladite pression P_{D}, une certaine quantité de fluide Q_{F} s'échappe de la chambre de pression vers l'extérieur du régulateur, ce qui induit une baisse de la pression P_{I} dans la chambre de pression, de manière telle que, en fin de perfusion, le ressort se comprimant de plus en plus par suite du déplacement de la plaque mobile et la pression du ressort P_{R} augmentant donc, le piston redescend dans la chambre de pression en refermant progressivement lesdits orifices, ce qui entraîne une diminution de la fuite Q_{F} et par conséquent une augmentation de la pression P_{I} dans la chambre de pression et dans le ballonnet.

D'autres caractéristiques et avantages de l'appareil selon l'invention apparaîtront par la description détaillée des formes de réalisation de l'invention données ci-dessous en relation avec les dessins annexés.

### Brève description des figures

Dans les dessins, la figure 1 représente un premier type d'appareil autonome de perfusion de la technique antérieure, dans lequel la présente invention peut être appliquée.

La figure 2 représente un deuxième type de dispositif autonome de perfusion de la technique antérieure, dans lequel la présente invention peut être appliquée.

La figure 3 montre, à titre d'exemple, un dispositif de régulation du débit installé selon l'invention dans l'appareil autonome de perfusion de la figure 1.

La figure 4 illustre une variante du régulateur de débit utilisé selon la figure 3.

Il va de soi que ces dessins ne constituent que des représentations schématiques simplifiées de l'appareil de l'invention, sans échelle particulière et dans lesquelles on n'a reproduit que les éléments indispensables pour la compréhension claire de l'invention. Des éléments identiques ou équivalents sont désignés par les mêmes repères numériques dans toutes les figures.

### Description de formes d'exécution préférées de l'invention

La description détaillée qui suit porte sur des formes de réalisation actuellement préférées de l'invention, et elle ne doit pas être considérée comme une limitation de l'invention à ces seules formes de réalisation.

La figure 1 illustre schématiquement un premier type d'appareil autonome de perfusion faisant partie de l'état de la technique précité. Dans un boîtier, il comporte une plaque inférieure 1 mobile verticalement, couplée à un mécanisme élastique lui-même composé de leviers croisés 2, 3. Une plaque supérieure 4 formant couvercle à rabattre permet d'exercer sur une poche 5 une force F qui, par l'intermédiaire de la poche 5, repousse la plaque mobile 1 vers le bas en armant le mécanisme élastique 2, 3. Le mécanisme élastique 2, 3 est mis sous tension, ou armé, au moyen de ressorts 6. La poche 5 ainsi comprimée est prête pour une perfusion.

La figure 2 illustre schématiquement un deuxième type d'appareil autonome de perfusion faisant également partie de l'état de la technique précité. Cet appareil comporte, dans un boîtier, une plaque supérieure 1 mobile verticalement, couplée à un mécanisme élastique lui-même composé de leviers croisés 2, 3. A la différence de l'appareil de la figure 1, le mécanisme élastique 2, 3 est ici placé au-dessus de la plaque mobile 1, entre celle-ci et une paroi supérieure fixe 7 du boîtier. Une poche de perfusion 5, représentée en traits interrompus, peut être déposée sur une plaque inférieure 8, fixe en direction verticale mais coulissant horizontalement à la manière d'un tiroir 4. Une pression de perfusion est produite en appliquant une force F au moyen de la plaque mobile 1, qui est déplacée verticalement vers le bas. Le mécanisme élastique 2, 3 est également entraîné par des ressorts 6 pour déplacer la plaque mobile 1.

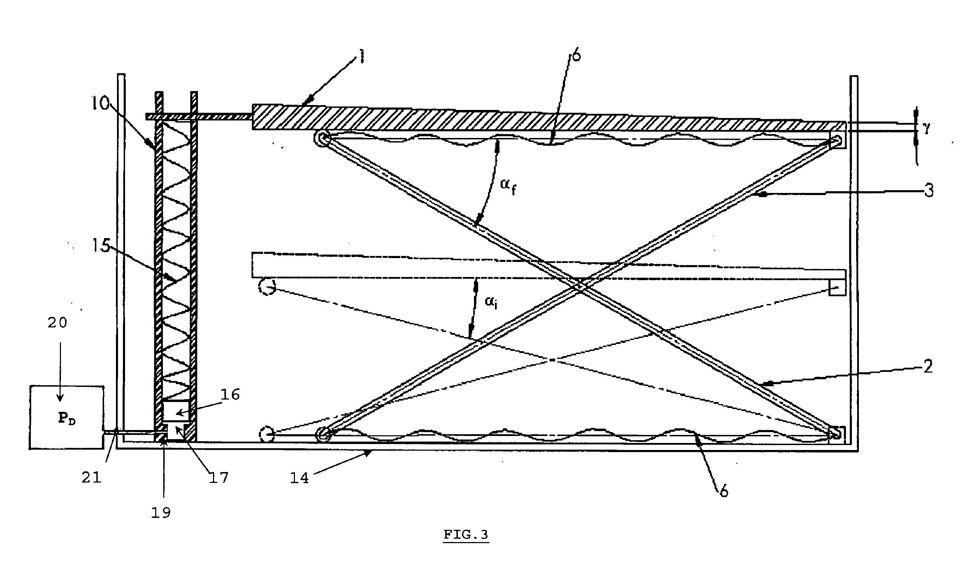

On a représenté dans la figure 3 un appareil autonome de perfusion, connu en soi, équipé, selon une première modalité de l'invention, d'un régulateur de débit basé sur un ressort hélicoïdal de compression 15 couplé à la plaque mobile 1. Cette plaque mobile 1 est représentée ici d'une part en position haute, ou position de repos, en traits pleins, et d'autre part en position basse, ou position d'armement, en traits interrompus. La position haute correspond à l'état déployé du mécanisme élastique 2, 3, c'est-à-dire à la fin de la perfusion. Dans cette position, l'angle α_{f} vaut de préférence 28 à 35°. La position basse correspond à l'état replié du mécanisme élastique 2, 3, c'est-à-dire au début de la perfusion (système armé). Dans cette position, l'angle αᵢ vaut de préférence 8 à 15°.

Comme on peut également le voir dans la figure 3, la face supérieure de la plaque mobile 1 présente une inclinaison, dans le sens de la vidange de la poche, avec un angle d'inclinaison γ inférieur à 10° par rapport à l'horizontale. Une telle inclinaison permet d'améliorer encore le taux de vidange de la poche, qui peut ainsi atteindre 99 %, par l'action de la gravité et de la force tangentielle définie plus haut.

Le régulateur de débit 10, représenté sur la figure 3, couplé selon l'invention à la plaque mobile 1, comporte essentiellement un ressort de compression hélicoïdal 15 taré de façon à augmenter la force de pression en fin de perfusion. Le ressort hélicoîdal 15 a son axe perpendiculaire à la plaque mobile. Une extrémité du ressort 15 est couplée à la plaque mobile 1 et l'autre extrémité est reliée indirectement à un appui fixe 14 sur le boîtier via une douille dans laquelle coulisse un piston 16, attaché d'une part à l'extrémité inférieure du ressort 15 et délimitant d'autre part une chambre de pression 17 formée dans la douille. Ladite chambre de pression 17 est reliée en circuit fermé, par une conduite 21 aboutissant à un orifice 19, à une source de pression constante 20 (P_{D}), par exemple un mini-compresseur, qui exerce sur le piston 16 une force de pression qui compense la diminution progressive de l'effort du ressort 15 sur la plaque mobile 1 au cours de la perfusion. En effet le ressort 15 est comprimé lors de l'armement du système. Le piston 16 vient se positionner à fond de butée dans la douille. Lors de la perfusion, la plaque mobile 1 remonte, sous l'effet du mécanisme élastique 2,3. En même temps le ressort de compression 15 a tendance à se détendre, ce qui réduit progressivement l'effort supplémentaire appliqué à la plaque mobile 1 par le ressort 15. Mais, comme le ressort 15 est soumis, via le piston 16, à la force de pression générée par P_{D}, il maintient en fin de perfusion un effort sur la plaque mobile 1 qui contrecarre la forte diminution de prssion, lorsque seul le mécanisme élastique lié au pantographe est présent. Lorsqu'on réarme le système, le fluide créant la pression dans la douille est refoulé par l'orifice 19 vers la source de pression 20.

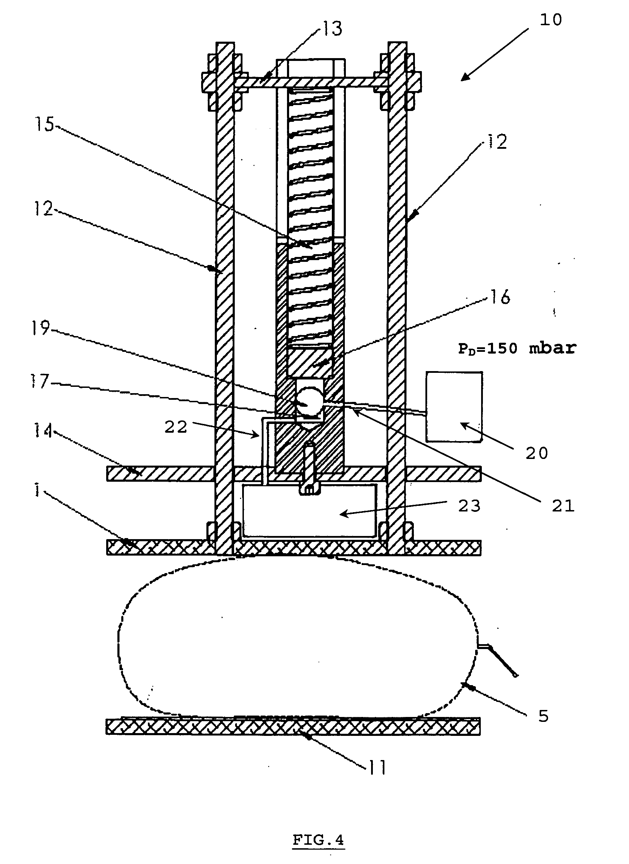

Dans la figure 4, on a représenté une variante du régulateur de débit 10 selon l'invention.

Selon cette variante, le régulateur 10 est couplé à la plaque supérieure mobile 1, qui se déplace en direction d'une plaque inférieure fixe 11 sous l'action d'un mécanisme élastique, connu en soi et non représenté ici (par ex. un pantographe). Dans cette configuration, l'angle γ défini plus haut mais non représenté ici, serait bien entendu prévu sur la plaque inférieure fixe 11.

Dans la forme de réalisation illustrée ici, la plaque mobile 1 se déplace donc vers le bas en direction d'une plaque fixe inférieure 11. L'invention n'est cependant pas limitée à cette forme de réalisation particulière. Au contraire, elle s'applique également, moyennant une adaptation simple, à un appareil dans lequel la plaque mobile 1 se déplace vers le haut en direction d'une plaque fixe supérieure.

Dans la figure 4, le régulateur 10 comprend un cadre rigide essentiellement rectangulaire composé de deux tiges 12 parallèles l'une à l'autre fixées perpendiculairement à la plaque mobile 1 par une première extrémité et reliées l'une à l'autre, à leur autre extrémité, par une traverse 13 parallèle à la plaque mobile 1. Un appui fixe 14 est prévu dans le boîtier, non représenté, de l'appareil de perfusion, entre la plaque mobile 1 et la traverse 13.

Un ressort hélicoïdal 15 est monté entre la traverse 13 et l'appui fixe 14, avec son axe longitudinal parallèle aux tiges 12 et donc perpendiculaire à la plaque mobile 1. A son extrémité supérieure, le ressort 15 prend appui de façon conventionnelle sur la traverse 13. A son extrémité opposée, le ressort 15 est relié indirectement à l'appui fixe 14 par une douille, non repérée, dans laquelle il est prévu un piston 16, attaché d'une part à l'extrémité inférieure du ressort 15 et délimitant d'autre part une chambre de pression 17 formée dans la douille. La chambre de pression 17 est reliée, via un connecteur 19, d'une part par une première conduite 21 à la sortie d'une source de pression 20 (P_{D}), par exemple un mini-compresseur, et d'autre part par une seconde conduite 22 à un ballonnet gonflable 23, jouant le rôle de vérin souple, en contact dans le boîtier entre la plaque d'appui 14 et la plaque mobile 1.

Le déplacement de la plaque mobile 1 pendant la perfusion, sous l'action de la force F appliquée par ledit mécanisme élastique non représenté, entraîne la compression du ressort 15, qui exerce dès lors une pression P_{R} dirigée vers le bas sur le piston 16. Cette pression P_{R} augmente progressivement au cours de la perfusion.

La pression P_{D} fournie par la source de pression est injectée par le connecteur 19 dans la chambre de pression 17 et agit vers le haut sur le piston 16 ; cette pression P_{D} est toujours supérieure à la pression P_{R} exercée en sens contraire par le ressort 15. En même temps, le ballonnet 23 se gonfle.

La différence de pression (P_{D} - P_{R}) provoque le déplacement du piston 16 vers le haut, libérant ainsi des orifices calibrés (non représentés) dans le régulateur 10, par lesquels une certaine quantité de fluide de pression (par ex. de l'air comprimé) Q_{F} s'échappe de la chambre de pression 17 vers l'environnement extérieur. Il en résulte une baisse de la pression qui s'établit à la valeur P_{I} dans la chambre de pression 17 et dans le ballonnet 23. Par exemple, si P_{D} vaut 150 mbar, la pression dans le ballonnet chute à P_{I} = 120 mbar.

En fin de perfusion, le ressort 15 se comprime de plus en plus par suite du déplacement de la plaque mobile 1 ; la pression P_{R} augmente, le piston 16 redescend vers la chambre de pression 17 et obture progressivement les orifices d'échappement, ce qui entraîne une diminution de la fuite Q_{F} et par conséquent une augmentation de la pression P_{I} et donc de la pression dans le ballonnet qui, dans l'exemple, retourne à 150 mbar. Cette augmentation de la pression P_{I} s'oppose à la diminution de pression dans la poche 5 due à la non-linéarité de la tangente α au cours de la perfusion.

Un appareil autonome de perfusion équipé d'un régulateur de débit selon l'invention permet ainsi d'atteindre une constance de la pression, donc du débit, de 95 à 99 %.

De plus, le taux de vidange des poches est amélioré, jusqu'à atteindre 99 %, grâce à la pente d'angle γ de 3 à 5° prévue sur la face supérieure de la plaque inférieure.

Le dispositif de l'invention peut encore comporter des modules connus en soi, qui permettent de mieux contrôler le déroulement et le suivi de la perfusion.

A cet égard, un premier module relatif à l'état de la perfusion peut comprendre un ou plusieurs des systèmes suivants :
- un système composé d'une roue codeuse et d'un capteur optoélectronique détectant la variation de hauteur de la plaque mobile ;
- un système permettant de signaler l'état de la perfusion ;
- un système de signalisation sonore et/ou visuelle de la fin de la perfusion ;
- un système de signalisation sonore et/ou visuelle de tout défaut survenant pendant la perfusion : perte de pression, bouchage de la ligne de perfusion, et analogues.

Un deuxième module comprend des moyens permettant de contrôler le débit du liquide de perfusion, en particulier à aide d'un débitmètre massique, avec un système d'affichage et d'alarme en cas de variation du débit au-delà de limites prédéterminées.

Un troisième module peut être consacré au contrôle de la perfusion et à la régulation du débit du liquide de perfusion. Ce module comprend par exemple un débitmètre massique pour contrôler le débit, ainsi qu'un système de régulation du débit à l'aide d'une micro-vanne pilotée par un servomoteur et agissant sur la section d'un tube compressible. Ce module comporte avantageusement des moyens d'affichage du débit ainsi qu'un système d'alarme en cas de variation du débit au-delà de limites prédéterminées.

L'invention n'est de toute manière pas limitée aux formes de réalisation décrites et illustrées ici. Au contraire, elle englobe les modifications que l'homme de métier pourrait y apporter, notamment en ce qui concerne le positionnement et la configuration du régulateur, et qui entrent dans le cadre des revendications annexées.

## Revendications

1. Appareil autonome de perfusion qui comporte un boîtier et des moyens pour mettre une poche de perfusion (5) sous pression entre une plaque fixe et une plaque mobile (1) essentiellement parallèle à ladite plaque fixe et entraînée perpendiculairement à celle-ci par un mécanisme élastique (2, 3, 6) entre une position d'armement et une position de repos, **caractérisé en ce qu'**il est muni d'un régulateur (10) de la pression exercée sur la poche (5) par ladite plaque mobile (1), comprenant un ressort (15) ayant un axe perpendiculaire à la plaque mobile (1) et dont une extrémité est couplée à la plaque mobile (1) et l'autre extrémité est reliée indirectement à un appui fixe (14) via une douille contenant un piston (16), attaché d'une part à l'extrémité inférieure du ressort (15) et délimitant d'autre part une chambre de pression (17) formée dans la douille, ladite chambre de pression (17) étant reliée en circuit fermé, par une conduite (21) aboutissant à un orifice (19), à une source de pression P_{D} (20), qui exerce sur le piston (16) une force de pression qui compense la diminution de l'effort du ressort (15) sur la plaque mobile (1) au cours de la perfusion, ce qui par conséquent compense la diminution de la pression appliquée à la plaque mobile (1) par ledit mécanisme élastique (2, 3, 6) .

2. Appareil autonome de perfusion selon la revendication 1, **caractérisé en ce que** ledit ressort est un ressort de compression hélicoïdal.

3. Appareil autonome de perfusion selon la revendication 1 ou 2, **caractérisé en ce que** la face supérieure de la plaque portant la poche de perfusion (5) est inclinée par rapport à un plan horizontal, en direction de la sortie de la poche de perfusion (5).

4. Appareil autonome de perfusion selon la revendication 3, **caractérisé en ce que** l'angle d'inclinaison γ de ladite face supérieure est inférieur à 10°, et est de préférence compris entre 3° et 5°.

5. Appareil autonome de perfusion selon l'une quelconque des revendications précédentes, **caractérisé en ce qu'**il comporte des moyens de verrouillage de la plaque mobile dans sa position d'armement.

6. Appareil autonome de perfusion selon l'une quelconque des revendications précédentes, **caractérisé en ce qu'**il comporte des moyens pour déverrouiller ladite plaque mobile en vue de permettre son mouvement vers sa position de repos.

7. Appareil autonome de perfusion selon l'une quelconque des revendications précédentes, **caractérisé en ce qu'**il comporte des moyens de surveillance du degré de vidange de la poche pendant la perfusion.

8. Appareil autonome de perfusion selon la revendication 7, **caractérisé en ce que** lesdits moyens de surveillance comprennent au moins une fenêtre graduée, éventuellement disposée dans un panneau vertical de l'appareil et s'étendant sur une hauteur au moins égale à la course maximale de la plaque mobile.

9. Appareil autonome de perfusion selon l'une quelconque des revendications précédentes, **caractérisé en ce que** le mécanisme élastique est du type à leviers croisés (2, 3) et à ressorts (6) et **en ce que** l'angle α d'ouverture du mécanisme élastique varie entre une valeur initiale αᵢ de 8 à 15° et une valeur finale α_{f} de 28 à 35°.

10. Appareil autonome de perfusion selon l'une quelconque des revendications précédentes, **caractérisé en ce qu'**il est logé dans un boîtier essentiellement parallélépipédique, présentant une longueur de 150 à 300 mm, une largeur de 100 à 200 mm et une hauteur de 40 à 80 mm.

11. Appareil autonome de perfusion qui comporte un boîtier et des moyens pour mettre une poche de perfusion (5) sous pression entre une plaque fixe et une plaque mobile (1) essentiellement parallèle à ladite plaque fixe et entraînée perpendiculairement à celle-ci par un mécanisme élastique entre une position d'armement et une position de repos, **caractérisé en ce qu'**il est muni d'une source extérieure de pression (20) alimentant, via un régulateur de pression (10), un vérin souple (23) appuyant sur la plaque mobile (1), ces éléments étant configurés et agencés pour augmenter au cours de la perfusion la pression exercée par le vérin souple (23) sur la poche (5) via la plaque mobile (1), cette augmentation de pression s'opposant à la diminution de la pression appliquée à la plaque mobile (1) par ledit mécanisme élastique et par conséquent à la diminution de la pression de sortie de la poche (5) au cours de la perfusion.

12. Appareil autonome de perfusion selon la revendication 11, **caractérisé en ce que** ledit régulateur de pression (10) comprend :
- un cadre rigide essentiellement rectangulaire composé de deux tiges (12) parallèles l'une à l'autre, fixées perpendiculairement à la plaque mobile (1) par une première extrémité et reliées l'une à l'autre, à leur seconde extrémité, par une traverse (13) parallèle à la plaque mobile (1) ;
- un ressort hélicoïdal (15) reliant à son extrémité supérieure la traverse (13) et à son autre extrémité un appui fixe (14) prévu dans le boîtier de l'appareil de perfusion, entre la plaque mobile (1) et la traverse (13), l'axe longitudinal du ressort (15) étant parallèle aux tiges (12) et donc perpendiculaire à la plaque mobile (1), la liaison du ressort (15) à l'appui fixe (14) étant réalisé indirectement via une douille contenant un piston (16), attaché d'une part à l'extrémité inférieure du ressort (15) et délimitant d'autre part une chambre de pression (17) formée dans la douille ;
- une première conduite reliant la chambre de pression (17) à la sortie de la source de pression P_{D} (20) et une seconde conduite (22) reliant la chambre de pression (17) au vérin souple, qui comprend un ballonnet gonflable (23), appuyant sur la plaque mobile (1) ;
- des orifices calibrés dans le régulateur (10), par lesquels, lors du déplacement du piston (16) vers le haut sous l'effet de ladite pression P_{D} (20), une certaine quantité de fluide Q_{F} s'échappe de la chambre de pression (17) vers l'extérieur du régulateur (10), ce qui induit une baisse de la pression P_{I} dans la chambre de pression (17), de manière telle que, en fin de perfusion, le ressort (15) se comprimant de plus en plus par suite du déplacement de la plaque mobile (1) et la pression du ressort P_{R} augmentant donc, le piston (16) redescend dans la chambre de pression (17) en refermant progressivement lesdits orifices, ce qui entraîne une diminution de la fuite Q_{F} et par conséquent une augmentation de la pression P_{I} dans la chambre de pression (17) et dans le ballonnet (23).
